(19) Europäisches Patentamt
European Patent Office
Office européen des brevets

(11) Veröffentlichungsnummer: **0 022 750**
**B1**

(12) **EUROPÄISCHE PATENTSCHRIFT**

(45) Veröffentlichungstag der Patentschrift: **18.05.83**

(51) Int. Cl.³: **C 07 D 285/12,** A 01 N 47/30, A 01 N 47/32, A 01 N 47/38

(21) Anmeldenummer: **80810222.2**

(22) Anmeldetag: **07.07.80**

# (54) 1,3,4-Thiadiazolyloxy-phenylharnstoffe, deren Herstellung und sie enthaltende herbizide Mittel.

(30) Priorität: **13.07.79 CH 6554/79**

(43) Veröffentlichungstag der Anmeldung: **21.01.81 Patentblatt 81/3**

(45) Bekanntmachung des Hinweises auf die Patenterteilung: **18.05.83 Patentblatt 83/20**

(84) Benannte Vertragsstaaten: **BE CH DE FR GB IT LI NL**

(56) Entgegenhaltungen:
**EP-A-0 001 328**
**DE-A-2 853 791**

(73) Patentinhaber: **CIBA-GEIGY AG, Patentabteilung Postfach, CH-4002 Basel (CH)**

(72) Erfinder: **Pissiotas, Georg, Dr., Breslauerstrasse 8, D-7850 Lörrach (DE)**
Erfinder: **Rohr, Otto, Dr., Kilbertweg 19, CH-4106 Therwil (CH)**
Erfinder: **Kristinsson, Haukur, Dr., Kreuzackerweg 19, CH-4103 Bottmingen (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

# 1,3,4-Thiadiazolyloxyphenylharnstoffe, deren Herstellung und sie enthaltende herbizide Mittel

Die vorliegende Erfindung betrifft neue 1,3,4-Thiadiazolyloxyphenylharnstoffe, mit herbizider Wirkung, deren Herstellung, sie enthaltende Mittel sowie deren Verwendung zur selektiven Unkrautbekämpfung in verschiedenen Kulturen, wie z.B. Getreide, Mais, Reis, Baumwolle, Zuckerrübe und Soja.

Herbizid wirkende Phenylharnstoffe sind längst bekannt und eingeführt. Durch ihre Verbreitung konnten Kulturen, wie Getreide, einerseits weitgehend von Unkraut befreit werden, anderseits ist es zu einer Proliferation von Unkräutern gekommen, die gegenüber solchen Herbiziden resistenter waren.

Durch die vorliegende Erfindung sollen einerseits auch bisher noch resistente Unkräuter bekämpft werden, anderseits können Kulturen geschont werden, wie z.B. Soja, die gegenüber der Phytotoxizität der meisten bekannten Herbizide nicht resistent genug waren.

Die neuen 1,3,4-Thiadiazolyloxyphenylharnstoffe entsprechen der Formel I

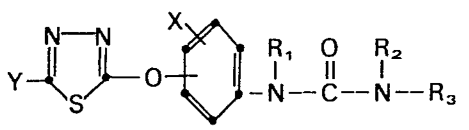
(I)

worin

X Wasserstoff, Halogen oder Trifluormethyl,

Y Wasserstoff, $C_1$ bis $C_6$-Alkyl, gegebenenfalls durch Sauerstoff unterbrochen oder durch Halogen oder Cyan substituiert, $C_3$ bis $C_6$-Cycloalkyl, Phenyl, gegebenenfalls substituiert durch Halogen oder $C_1$ bis $C_4$-Alkyl, $C_1$ bis $C_4$-Alkylthio, $C_1$ bis $C_4$-Alkoxy, $C_1$ bis $C_4$-Alkylsulfinyl oder Alkylsulfonyl oder eine Sulfamoylgruppe $-SO_2NR_2R_3$,

$R_1$ Wasserstoff oder $C_1$ bis $C_4$-Alkyl,

$R_2$ Wasserstoff, $C_1$ bis $C_6$-Alkyl, gegebenenfalls durch Sauerstoff unterbrochen oder durch Halogen oder Cyan substituiert, $C_3$ bis $C_6$-Alkenyl oder Alkinyl, $C_3$ bis $C_6$-Cycloalkyl,

$R_3$ dasselbe wie $R_2$ oder $C_1$ bis $C_6$-Alkoxy, oder

$R_2$ und $R_3$ zusammen mit dem Stickstoff, an das sie gebunden sind, auch einen 5-6gliedrigen Heterocyclus, der noch ein Sauerstoff- oder Schwefelatom oder eine gegebenenfalls durch $C_1$ bis $C_3$-Alkyl substituierte Iminogruppe enthalten kann.

Die unter Y und $R_1$, $R_2$ und $R_3$ definierten Alkylreste können sowohl geradkettig wie verzweigt sein. Als Reste Y kommen hauptsächlich in Frage: Wasserstoff, Methyl, Äthyl, n-Propyl, Isopropyl, n-Butyl, s-Butyl, Isobutyl, tert.-Butyl, Amyl, Isoamyl, Neopentyl, n-Hexyl sowie Hex-2-yl, Methoxymethyl, Methoxyäthyl, Cyanomethyl, Cyanoäthyl, 2-Cyanopropyl, Trifluormethyl, Trichlormethyl, Cyclopropyl, Cyclopropylmethyl, Cyclobutyl, Cyclopentyl, Cyclohexyl, Methylthio, Äthylthio, n-Propylthio, Isopropylthio, n-Butylthio, Isobutylthio, s-Butylthio, t-Butylthio, Methylsulfinyl, Äthylsulfinyl, n-Propylsulfinyl, Isopropylsulfinyl, n-Butylsulfinyl, Isobutylsulfinyl, s-Butylsulfinyl, t-Butylsulfinyl, Methylsulfonyl, Äthylsulfonyl, n-Propylsulfonyl, Isopropylsulfonyl, n-Butylsulfonyl, Isobutylsulfonyl, s-Butylsulfonyl, t-Butylsulfonyl sowie Dimethylsulfamoyl und Diäthylsulfamoyl. Bevorzugt als Rest $R_1$ ist Wasserstoff oder Methyl, als Reste $R_2$ und $R_3$ sind Methyl und Methoxy.

Die Verbindungen der Formel I weisen ausgesprochen selektivherbizide Eigenschaften im allgemeinen auf und erweisen sich als besonders vorteilhaft zum Bekämpfen von Unkräutern in Kulturen von Nutzpflanzen, insbesondere in Kulturen von Soja, Baumwolle, Getreide, Mais und Zuckerrüben.

Bei genügend grosser Aufwandmenge ist jedoch auch totalherbizide Wirkung vorhanden. Die Aufwendung kann sowohl im Vorauflauf, wie im Nachauflaufverfahren, vorgenommen werden. Dabei können die Aufwandmengen in weiten Grenzen schwanken, z.B. zwischen 0,1 bis 10 kg Wirkstoff/ha, vorzugsweise werden jedoch 0,5 bis 5 kg Wirkstoff/ha, eingesetzt.

Phenylharnstoffe werden seit langem als Herbizide verwendet, siehe beispielsweise die DE-PS Nr. 935165 oder Nr. 968273. Phenoxyphenylharnstoffe, welche sich gut zur Bekämpfung von Unkräutern eignen, wenn entweder allein oder zusammen mit einem Phenoxypropionsäureherbizid angewandt, sind ebenfalls bekannt, siehe die DE-PS Nr. 1142251 und die EP-A Nr. 0001328. Kürzlich sind Naphthyloxyphenylharnstoffe bekannt geworden, welche sich zur selektiven Unkrautbekämpfung in Sojakulturen eignen. Die erfindungsgemässen 1,3,4-Thiadiazolyloxyphenylharnstoffe eignen sich in überraschend geringen Aufwandmengen zur selektiven Unkrautbekämpfung in diversen Nutzpflanzenkulturen.

Die beste Wirkung wurde mit Verbindungen erreicht, die folgenden strukturellen Gruppen entsprechen:

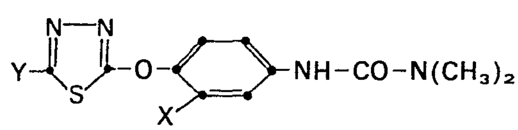
(Ia)

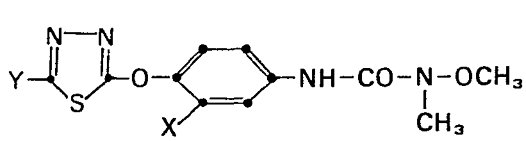
(Ib)

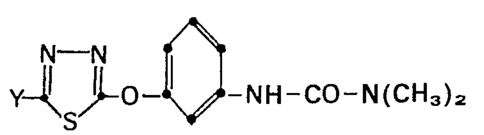
(Ic)

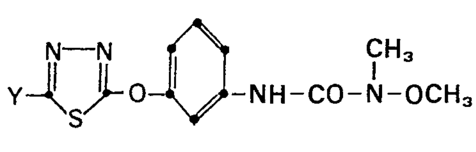
(Id)

In diesen Formeln haben X und Y die oben gegebene Bedeutung. Besonders günstig wirkten diejenigen, in denen Y die Trifluormethyl-, Isopropyl-, Isobutyl-, sek.-Butyl-, tert.-Butyl-, Isoamyl-, Cyclopropyl-, Cyclopropylmethyl-, Methoxymethyl-, 1-Cyanopropyl-, Methylsulfonyl, Isopropylsulfonyl oder Dimethylsulfamoylgruppe darstellte sowie die Verbindungen, in denen X die Trifluormethylgruppe oder Chlor und Y die tert.-Butylgruppe darstellen.

Die erfindungsgemässen Mittel enthalten ausser dem Wirkstoff der Formel I einen geeigneten Träger und/oder andere Zuschlagstoffe. Geeignete Träger und Zuschlagstoffe können fest oder flüssig sein und entsprechen den in der Formulierungstechnik üblichen Stoffen, wie z.B. natürlichen oder regenerierten mineralischen Stoffen, Lösungs-, Verdünnungs-, Dispergier-, Emulgier-, Netz-, Haft-, Verdickungs-, Binde- und/oder Düngmitteln.

Zur Verwendung in herbiziden Mitteln kann die Verbindung der Formel I als Stäubemittel, Emulsionskonzentrat, als Granulat, Dispersion oder auch als Lösung oder Aufschlämmung in üblicher Formulierung verarbeitet werden.

Die Verbindungen der Formel I zerfallen im Boden nach relativ kurzer Zeit in stickstoffreiche Zersetzungsprodukte, welche der Pflanzenernährung zuträglich sind.

Zur Verbreiterung des Wirkungsspektrums oder zur Erreichung eines erwünschten synergistischen oder auch antagonistischen Effektes können diese Verbindungen auch gemeinsam mit bekannten herbiziden, pestiziden oder fungiziden Verbindungen eingesetzt werden. Als Kombinationspartner kommen beispielsweise herbizid wirkende Verbindungen folgenden Typs in Frage:

Phenoxyaliphatische Säuren, substituierte Harnstoffe, Triazine, Phenole, Nitrile, Bipyridinverbindungen, substituierte Benzoesäuren, halogenierte aliphatische Säuren, Carbamate, Thiocarbamate, Chloracetamide, Diazine, Benzofuran oder Herbizide auf Arsenbasis.

Phenoxyaliphatische Säuren bestehen im allgemeinen aus Alkyl- und/oder halogensubstituierten phenoxyaliphatischen Säuren und ihren Salzen, z.B. mit Alkalimetallen, Aminen oder Alkanolaminen, ferner deren funktionelle Derivate, wie z.B. die Ester und Amide. Diese Verbindungen mögen eine Aktivität besitzen, wie sie kommerziellen Produkten eigen ist, oder sie mögen auch nur geringe herbizide Wirkung aufweisen. Als Beispiele von solchen substituierten phenoxyaliphatischen Säuren seien genannt: 2,4-Dichlorphenoxyessigsäure, 2-(2,4-Dichlorphenoxy)propionsäure, 2-Methyl-4-chlorphenoxyessigsäure, 2,4,5-trichlorphenoxyessigsäure, $\gamma$-2,4-Dichlorphenoxybuttersäure, $\gamma$-2-Methyl-4-chlorphenoxypropionsäure, 2-[4-(2,4-Dichlorphenoxy)phenoxy]-propionsäure und 2-[4-(4-Chlorphenoxy)phenoxy]propionsäure.

Die substituierten Harnstoffe umfassen tri- und tetrasubstituierte Harnstoffe, wie z.B. N'-(3-Chlor-4-methoxyphenyl)-N,N-dimethylharnstoff, N'-(3-Chlor-4-methylphenyl)-N,N-dimethylharnstoff, N'-(p-Chlorphenyl)-N,N-dimethylharnstoff, N-Butyl-N'-(3,4-dichlorphenyl)-N-methylharnstoff, N'-p-Chlorphenyl-O,N,N-trimethylisoharnstoff, N'-p-Chlorphenyl-N-methoxy-N-methylharnstoff, N,N-Dimethyl-N'-phenylharnstoff, 3-(4'-Bromphenyl)-1-methoxy-1-methylharnstoff, 1-(2'-Benzothiazolyl)-3-methylharnstoff, N,N-Dimethyl-N'-[4-(1-methyläthyl)phenyl]harnstoff, N'-(3,4-Dichlorphenyl)-N-methoxy-N-methylharnstoff, N,N-Dimethyl-N'-[3-trifluormethyl)phenyl]harnstoff, N'-(3,4-Dichlorphenyl)-N,N-dimethylharnstoff oder N'-[3-(1',1',2',2'-Tetrafluorethoxy)phenyl]-N,N-dimethylharnstoff.

Das Triazinherbizid umfasst bevorzugt 2-Chlor-4-(1-cyano-1-methylamin)-6-äthylamin-1,3,5-triazin oder aber eine Verbindung der Formel

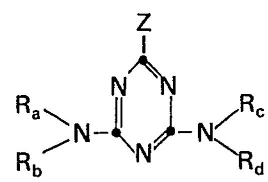

worin Z ein Halogenatom eine Alkoxy- oder Alkylthiogruppe und $R_a$ und $R_c$ gleich oder verschieden Wasserstoff oder Alkyl und $R_b$ und $R_d$ gleiche oder verschiedene Alkylgruppen bedeuten, wie z.B. 2-Chlor-4,6-bisäthylamino-1,3,5-triazin, 2-Chlor-4-äthylamino-6-isopropylamino-1,3,5-triazin oder 2,4-Bis(isopropylamino)-6-methylthio-1,3,5-triazin.

Die Phenolherbizide umfassen beispielsweise 4,6-Dinitro-o-kresol, 4,6-Dinitro-2-sek.-butylphenol oder Pentachlorphenol.

Die herbizide Nitrilverbindung umfasst im allgemeinen 3,5-Dijod-4-hydroxybenzonitril, 3,5-Dibrom-4-hydroxybenzonitril oder 2,6-Dichlorbenzonitril.

Als Bipyridinherbizidverbindung sei 1,1'-Dimethyl-4,4'-bipyridiniumdichlorid oder 1,1-Äthylen-2,2'-bipyridiniumdibromid genannt.

Substituierte Benzoesäureherbizide umfassen beispielsweise 2,3,6-Trichlorbenzoesäure, 2-Methoxy-3,6-dichlorbenzoesäure oder N-(1,1-Dimethylpropinyl)-3,5-dichlorbenzamid.

Als herbizide aliphatische Säuren seien Trichloressigsäure oder 2,2-Dichlorpropionsäure genannt. Carbamate mit herbizider Wirkung umfassen im allgemeinen N-(3-Chlorphenyl)carbamatisopropylester, N-(3-Chlorphenyl)carbamat-4'-chlorbut-2'-inylester, 3-(m-Tolylcarbamoyloxy)-phenylcarbamatmethylester, N-[3-(N-Äthyl-N-phenylcarbamoyl)phenylcarbamat]isopropylester oder D-N-Äthyl-2-(phenylcarbamoyl)propionsäureamid.

Als Herbizid wirkende Thiocarbamate seien genannt: S-Äthyl-N,N-dipropylthiocarbamat, S-Alkyl-N,N-diisobutylthiocarbamat, S-(2,3-Dichlorallyl)-N,N-diisopropylthiocarbamat, S-Äthyl-N-äthyl-N-cyclohexylthiocarbamat, S-Propylbutyläthylthiocarbamat oder S-(2,3,3-Trichlorallyl)-N,N-diisopropylthiocarbamat.

Geeignete Chloracetamidherbizide umfassen im allgemeinen N,N-Dichlordiallylacetamid, N-Isopropyl-2-chloracetanilid, N-Chloracetyl-N-(2,6-diäthylphenyl)glycineäthylester, N-(2,6-diäthylphenyl)-N-(2-methoxyphenyl)-N-(methoxy-1-methyläthyl)-2-chloracetamid oder N-Chloracetyl-N-(2-methyl-6-äthylphenyl)glycineisopropylester.

Diazinherbizide umfassen im allgemeinen S-Brom-6-methyl-3-sek.-butyluracil, 3-Cyclohexyl-5,6-trimethylenuracil, 5-Amino-4-chlor-2-phenyl-3-pyridazinon oder 1,2-Dihydropyridazin-3,6-dion.

Benzofuranherbizide sind beispielsweise Ethofumesat oder 2,3-Dihydro-3,3-dimethylbenzofuran-5-yläthansulfonat. Arsenhaltige Herbizide umfassen vor allem Salze, z.B. mono- oder di-Natriumsalze von Methanarsen- oder Cacodylsäure.

Weitere Herbizide, die zusammen mit den 1,3,4-Thiadiazolylphenylharnstoffen der Formel I verwendet werden, können umfassen: 1,2-Dimethyl-3,5-diphenylpyrazoliumion, N-Benzoyl-N-(3,4-dichlorphenyl)alaninäthylester, N-Isobutyl-2-oxo-1-imidazolidincarboxamid, Aminotriazol-2,3-dichlor-1,4-naphthochinon, 4-Amino-3,5,6-trichlorpicolinsäure, N,N-dimethyl-2,2-diphenylacetamid, 2,6-Dinitro-N,N-dipropyl-4-trifluoromethylanilin, N-Butyl-N-äthyl-2,6-dinitro-4-trifluormethylanilin, S,S,S-Tributylphosphortrithioate, 2-Äthoxy-2,3-dihydro-3,3-dimethyl-5-benzofuranylmethylsulfonat, 4-Chlor-2-oxobenzothiazolin-3-ylessigsäure, 3-Isopropyl-2,1,3-benzothiadiazinon-(4)-2,2-dioxyd, 3,5-Dibrom-4-hydroxybenzaldehyd, 2,4-Dinitrophenyloxim, 2-Chlor-3-(4-chlorphenyl)propionsäuremethylester, 2-Chloräthyltrimethylammoniumchlorid, 4-Methylsulfonyloxy-2-butyryl-m-chlorcarbanilat, 2-(N-benzoyl-3-chlor-4-fluoranilino)propionsäuremethylester, 2-Chlor-N-(1,3-dioxolan-2-ylmethyl)-2',6'-dimethylacetanilid, 2-Chlor-1-(3-äthoxy-4-nitrophenoxy)-4-trifluormethylbenzol, 2-[4-(2',4'-dichlorphenoxy)phenoxypropionsäuremethylester, 1,1,1-Trifluor-2'-methyl-4'-(phenylsulfonyl)methansulfonanilid, 4-Chlor-5-methylamino-2-(3-trifluormethylphenyl)-3-[2H]-pyridazinon, S-(2-Chlor-4-trifluormethylphenoxy)-2-nitrobenzoesäure, 1-Methyl-3-phenyl-S-(3-trifluormethylphenyl)-4-pyridon, 4-(Methylsulfonyl)-2,6-dinitro-N,N-dipropylanilin, 4-(2,4-Dichlorphenoxy)-2-methoxy-1-nitrobenzol, N-(Äthoxypropyl)-3,5-dimethyl-2,6-dinitroanilin, 2',4'-Dimethyl-5'-(trifluormethansulfonamid)acetanilid, Dimethyl-2,3,5,6-tetrachlorterephthalat, N-Cyclopropylmethylterephthalat, N-Cyclopropylmethyl-2,6-dinitro-N-propyl-4-trifluormethylanilin, N-(2-Chloräthyl)-2,6-dinitro-N-propyl-4-trifluormethylanilin, N',N'-Diäthyl-2,6-dinitro-4-trifluormethyl-m-phenylendiamin, N-sek.-Butyl-4-tert.-butyl-2,6-dinitroanilin, 2-(3,4-Dichlorphenyl)-4-methyl-1,2,4-oxadiazolidin-3,5-dion, 4-(Dipropylamino)-3,5-dinitrobenzolsulfonamid, 1-(3-Trifluormethylphenyl)-5,5-dimethyl-4-methoxycarbonylcyclohexan-1,3-dion, 2-(N-Äthoxybutyrimidoyl)-5-(2-äthylthiopropyl)-3-hydroxy-2-cyclohexen-1-on oder 2-[1-(2,5-Dimethylphenyl)äthylsulfonyl]pyridin-N-oxid.

Die Verbindungen der Formel I können auch zusammen mit einer herbizid antagonistischen Substanz (welche die Eigenschaften hat, die Herbizidtoleranz einer Kultur gegen ein bestimmtes Herbizid zu verbessern) angewandt werden, z.B. mit N,N-Diallyldichloracetamid, 4'-Chlor-2-(hydroxyimino)acetanilid, 1,8-Dinaphthoesäureanhydrid, α-(Cyanomethoximino)benzolessigsäurenitril oder 2,2-Dimethyl-3-dichloressigsäureoxazolidin.

Der Antagonist kann zusammen mit dem Wirkstoff der Formel I verwendet werden. Es ist jedoch vorteilhaft, ihn getrennt anzuwenden, zum Beispiel als Beizmittel für die Kultursaat. Das Gewichtsverhältnis von Herbizid zu Antagonist kann in den Grenzen 10:1 zu 1:4 schwanken.

Die Herstellung der Verbindungen der Formel I erfolgt nach an sich bekannten Methoden.

Gemäss einem ersten Verfahren werden die 1,3,4-Thiadiazolyloxyphenylharnstoffe der Formel I hergestellt, indem man in einem den Reaktionsteilnehmern gegenüber inerten organischen Lösungsmittel, in Gegenwart der säurebindenden Menge einer Base, einen Hydroxyphenylharnstoff der Formel II

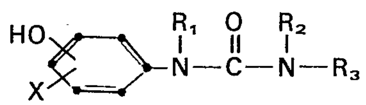 (II)

worin X, $R_1$, $R_2$ und $R_3$ die unter Formel I gegebene Bedeutung haben, mit einem 2-Halogen-1,3,4,-thiadiazolylderivat der Formel III umsetzt,

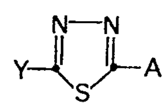 (III)

worin Y die unter Formel I gegebene Bedeutung hat und A Halogen oder einen $C_1$ bis $C_4$-Alkylsulfonylrest bedeutet.

Gemäss einem weiteren Verfahren werden die 1,3,4-Thiadiazolyloxyphenylharnstoffe der Formel I hergestellt, indem man ein 1,3,4-Thiadiazolyloxyanilin der Formel IV

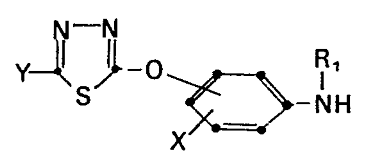 (IV)

worin Y und $R_1$ die unter Formel I gegebene Bedeutung haben, in einem den Reaktionsteilnehmern gegenüber inerten organischen Lösungsmittel, in Gegenwart der säurebindenden Menge einer Base, mit einem Carbamoylsäurehalogenid der Formel V umsetzt,

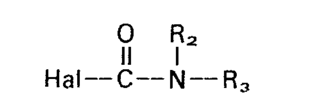 (V)

worin Hal Halogen bedeutet und $R_2$ und $R_3$ die unter Formel I gegebene Bedeutung haben.

Die 1,3,4-Thiadiazolyloxyphenylharnstoffe der Formel I, worin $R_1$ Wasserstoff bedeutet, können auch hergestellt werden, indem man ein 1,3,4-Thiadiazolyloxyanilin der Formel V, worin $R_1$ Wasserstoff bedeutet, mittels Phosgen zum 1,3,4-Thiadiazolyloxyphenylisocyanat der Formel VI umwandelt,

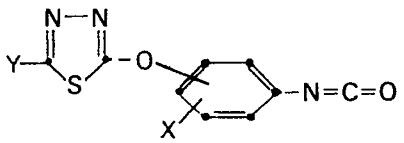
(VI)

worin X und Y die unter Formel I gegebene Bedeutung haben, und dieses in einem den Reaktionsteilnehmern gegenüber inerten organischen Lösungsmittel mit einem Amin der Formel VII umsetzt,

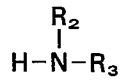
(VII)

worin $R_2$ und $R_3$ die unter Formel I gegebene Bedeutung haben.

Die Ausgangsstoffe der Formeln II und III sind bekannt und zum Teil im Handel erhältlich, dasselbe gilt für die Amine und das Carbamoylhalogenid der Formeln V und VII.

Das Anilin der Formel IV hergestellt durch Kondensation eines Nitrophenols der Formel VIII

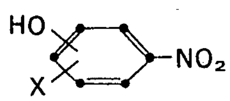
(VIII)

worin X die unter Formel I gegebene Bedeutung hat, mit dem 2-Halogeno-1,3,4- thiadiazolylderivat der Formel III und nachfolgender Reduktion der Nitrogruppe zum Amin. Das so erhaltene Anilin kann gegebenenfalls noch mit einem $C_1$ bis $C_4$-Alkyl(mono)halogenid alkyliert werden.

Diese Reaktionen und Umsetzungen werden in den Reaktionspartnern gegenüber inerten organischen Lösungsmitteln vorgenommen, wie Ketonen, höhersiedenden Alkanolen, Estern, Dimethylformamid, Dimethylsulfoxid, Dioxan etc. Es ist vorteilhaft, wenn die Lösungsmittel mit Wasser mischbar sind, da sich das Reaktionsprodukt durch Wasserzugabe ausfallen lässt.

Die Reaktionstemperatur beträgt 0-150° C, meistens wird alternierend zwischen Raumtemperatur und dem Siedepunkt des Reaktionsgemisches gearbeitet. Diese Reaktionen werden normalerweise unter gewöhnlichem Druck durchgeführt. Bei Herstellung grösserer Mengen sind jedoch Druckgefässe und höhere Drucke zu empfehlen.

Die Verbindungen der Formel I sind für Warmblüter wenig giftig, ihre Handhabung bedarf keiner vorsorglichen Massnahmen. Sie sind in den üblichen organischen Lösungsmitteln relativ gut und in Wasser schlecht löslich. Sie können durch Zugeben von Wasser zur Reaktionslösung leicht ausgefällt werden. Ihre Formulierung als flüssige herbizide Mittel gelingt nur mit Hilfe besonderer Lösungsvermittler und/oder Dispergiermittel.

Das folgende Beispiel soll die Herstellung der erfindungsgemässen Phenylharnstoffe der Formel I näher erläutern. Weitere in analoger Weise hergestellte Verbindungen sind in den anschliessenden Tabellen aufgeführt. Die Temperaturen sind in Celsiusgraden gegeben, Teile und Prozentangaben beziehen sich auf das Gewicht. Siedepunkte werden mit der Temperatur und, sofern sie nicht unter Atmosphärendruck gemessen wurden, mit dem in Millibar (mbar) gemessenen Druck angegeben.

*Beispiel*

N-[4-(5'-tert.-Butyl-1',3',4'-thiadiazolyl-2'-oxy)phenyl]-N',N'-dimethylharnstoff

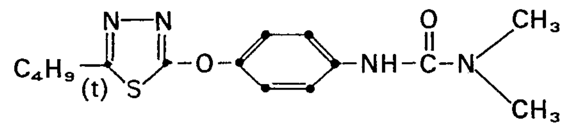

Ein Gemisch von 9 g N-(p-Hydroxyphenyl)-N',N'-dimethylharnstoff, 3,3 g pulverisiertem Kaliumhydroxid und 60 ml Dimethylsulfoxid wird ½ h lang auf 50° C erwärmt. Nach dem Abkühlen auf Raumtemperatur werden unter Rühren 8,9 g 2-Chlor-5-tert.-butyl-1,3,4-thiadiazol zugetropft. Nach zweistündigem Rühren bei 70° C wird das Reaktionsgemisch auf Eis/Wasser gegossen, das ausgefallene Endprodukt abgenutscht, mit Wasser gewaschen und im Vakuum getrocknet. Man erhält so 14 g des obigen Harnstoffes vom Schmelzpunkt 138-140° C.

Analog zu diesen Beispielen wurden folgende Verbindungen erhalten:

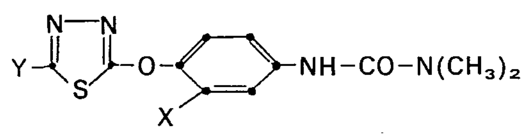

| Nr. | X | Y | physikalische Daten |
|---|---|---|---|
| 1.01 | H | $C(CH_3)_3$ | Smp. 138-140° |
| 1.02 | H | $CF_3$ | Smp. 138-140° |
| 1.03 | H | $CH_3$ | Smp. 118° |
| 1.04 | H | $C_2H_5$ | |
| 1.05 | H | $CH(CH_3)_2$ | Smp. 89-91° |
| 1.06 | H | $C_3H_{7n}$ | |
| 1.07 | H | $SC_4H_{9n}$ | Smp. 73-74° |
| 1.08 | H | $CH_2CH(CH_3)_2$ | |
| 1.09 | H | $CH(C_2H_5)_2$ | |
| 1.10 | H | $CH(CH_3)C_2H_5$ | |
| 1.11 | H | $CCl_3$ | |
| 1.12 | H | 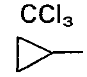 | Smp. 158° |
| 1.13 | H | 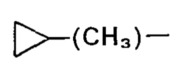 | |
| 1.14 | H | 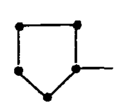 | |

| Nr. | X | Y | physikalische Daten |
|---|---|---|---|
| 1.15 | H | 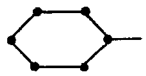 | |
| 1.16 | H | 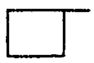 | |
| 1.17 | H | $SCH_3$ | |
| 1.18 | H | $SCH(CH_3)_2$ | |
| 1.19 | H | $SC(CH_3)_3$ | |
| 1.20 | H | $SO \cdot CH_3$ | |
| 1.21 | H | $SO_2CH_3$ | |
| 1.22 | H | $SO_2C_4H_{9n}$ | |
| 1.23 | H | $SO_2N(CH_3)_2$ | |
| 1.24 | H | 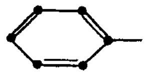 | Smp. 201-2° |
| 1.25 | H | 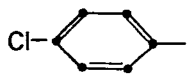 | Smp. 213-215° |
| 1.26 | H | 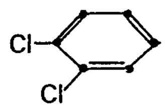 | |
| 1.27 | Cl | $CF_3$ | Smp. 158-9° |
| 1.28 | Cl | $CH_3$ | |
| 1.29 | Cl | 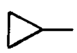 | |
| 1.30 | Cl | $C(CH_3)_3$ | Smp. 116° |
| 1.31 | Cl | $SO_2N(CH_3)_2$ | |
| 1.32 | Cl | $C(CH_3)_2CN$ | |
| 1.33 | Cl | $SO_2CH_3$ | |
| 1.34 | Cl | 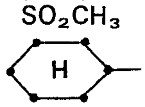 | |
| 1.35 | Cl | 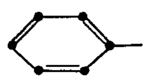 | |
| 1.36 | Cl | $CH_2CN$ | |
| 1.37 | Cl | $SO_2CH(CH_3)_2$ | |
| 1.38 | Cl | $SCH_3$ | |
| 1.39 | Cl | $SCH(CH_3)_2$ | |
| 1.40 | $CF_3$ | $C(CH_3)_3$ | |

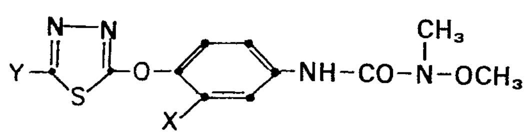

| Nr. | X | Y | physikalische Daten |
|---|---|---|---|
| 2.01 | H | $CF_3$ | Smp. 89-90° |
| 2.02 | H | $CH_3$ | Smp. 103-5° |
| 2.03 | H | 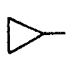 | $n_D^{29} = 1{,}5922$ |
| 2.04 | H | $SO_2N(CH_3)_2$ | |
| 2.05 | H | $CH(CH_3)_2$ | $n_D^{30} = 1{,}5820$ |
| 2.06 | H | $C(CH_3)_3$ | Smp. 95-96° |

| Nr. | X | Y | physikalische Daten |
|---|---|---|---|
| 2.07 | H | 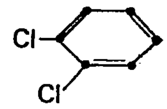 | Smp. 213-15° |
| 2.08 | H | 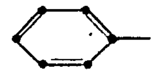 | Smp. 178-9° |
| 2.09 | H | $CH(C_2H_5)_2$ | |
| 2.10 | H | $SC_4H_{9n}$ | Smp. 78-79° |
| 2.11 | H | $CCl_3$ | |
| 2.12 | H | 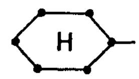 | |
| 2.13 | H | 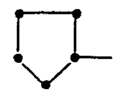 | |
| 2.14 | H | $SCH_3$ | |
| 2.15 | H | $SC_3H_{7n}$ | |
| 2.16 | H | $SO_2CH_3$ | |
| 2.17 | H | $SO_2C_4H_{9n}$ | |
| 2.18 | H | $CH_2CF_3$ | |
| 2.19 | H | $CH(CH_3)C_2H_5$ | |
| 2.20 | Cl | 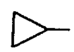 | |
| 2.21 | Cl | 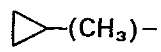 | |
| 2.22 | Cl | $SO_2N(CH_3)_2$ | |
| 2.23 | Cl | $CH_3$ | |
| 2.24 | Cl | $CH(CH_3)_2$ | |
| 2.25 | Cl | $C(CH_3)_3$ | Smp. 91-93° |
| 2.26 | Cl | $CH(C_2H_5)_2$ | |
| 2.27 | Cl | $CH(CH_3)C_2H_5$ | |
| 2.28 | Cl | $CF_3$ | |
| 2.29 | Cl | $CCl_3$ | |
| 2.30 | Cl | $CH_2CN$ | |
| 2.31 | Cl | $C(CH_3)_2CN$ | |
| 2.32 | Cl | $SCH_3$ | |
| 2.33 | Cl | $SC_2H_5$ | |
| 2.34 | Cl | $SCH(CH_3)_3$ | |
| 2.35 | Cl | $SC_4H_{9n}$ | |
| 2.36 | Cl | $SO_2C_3H_{7n}$ | |
| 2.37 | Cl | $SO_2C(CH_3)_3$ | |
| 2.38 | Cl | $SOCH_3$ | |
| 2.39 | $CF_3$ | $C(CH_3)_3$ | |

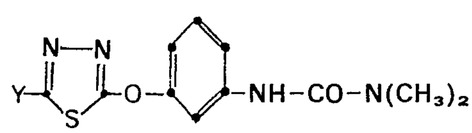

| Nr. | Y | physikalische Daten |
|---|---|---|
| 3.01 | $CF_3$ | Smp. 161-162° |
| 3.02 | $CH_3$ | Smp. 105-107° |
| 3.03 | 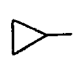 | $n_D^{30} = 1{,}5956$ |

| Nr. | Y | physikalische Daten |
|---|---|---|
| 3.04 | $C(CH_3)_3$ | Smp. 95-96° |
| 3.05 | $CH(CH_3)_2$ | $n_D^{29}$ = 1,5830 |
| 3.06 | $SO_2N(CH_3)_2$ | |
| 3.07 | 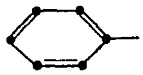 | Smp. 160-161° |
| 3.08 | 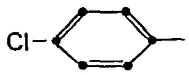 | |
| 3.09 | $SC_4H_{9n}$ | Smp. 95-96° |
| 3.10 | $CH(C_2H_5)_2$ | |
| 3.11 | $CH(CH_2)C_2H_5$ | |
| 3.12 | $CCl_3$ | |
| 3.13 | $CH(CH_3)CN$ | |
| 3.14 | $SCH_3$ | |
| 3.15 | $SC(CH_3)_3$ | |
| 3.16 | $SOCH_3$ | |
| 3.17 | $SO_2CH_3$ | |
| 3.18 | $SO_2C_3H_{7n}$ | |
| 3.19 | $SO_2C(CH_3)_3$ | |
| 3.20 | $SO_2CH(CH_3)C_2H_5$ | |
| 3.21 | $CH_2OCH_3$ | |

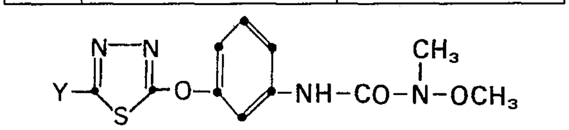

| Nr. | Y | physikalische Daten |
|---|---|---|
| 4.01 | $CF_3$ | Smp. 118-119° |
| 4.02 | $CH_3$ | Smp. 120-124° |
| 4.03 | 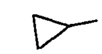 | $n_D^{30}$ = 1,5830 |
| 4.04 | 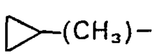 | |
| 4.05 | 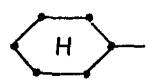 | |
| 4.06 | $C(CH_3)_3$ | Smp. 124-6° |
| 4.07 | $CH(CH_3)_2$ | $n_D^{30}$ = 1,5800 |
| 4.08 | $SO_2N(CH_3)_2$ | |
| 4.09 | 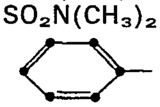 | Smp. 108-6° |
| 4.10 | 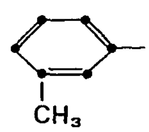 | Smp. 138-9° |
| 4.11 | $CH_2OCH_3$ | |
| 4.12 | $CH(C_2H_5)CN$ | |
| 4.13 | $SC_4H_{9n}$ | Smp. 98-99° |
| 4.14 | $SCH(CH_3)_2$ | |
| 4.15 | $SOC_4H_{9n}$ | |
| 4.16 | $SOC(CH_3)_3$ | |
| 4.17 | $SO_2CH_3$ | |
| 4.18 | $SO_2CH(CH_3)_2$ | |
| 4.19 | $SO_2CH(CH_3)C_2H_5$ | |
| 4.20 | $CCl_3$ | |

Die neuen Wirkstoffe der Formel I sind stabile Verbindungen, welche in üblichen organischen Lösungsmitteln, wie Alkoholen, Äthern, Ketonen, Dimethylformamid, Dimethylsulfoxyd etc., löslich sind.

Die Herstellung erfindungsgemässer Mittel erfolgt in an sich bekannter Weise durch inniges Vermischen und Vermahlen von Wirkstoffen der allgemeinen Formel I mit geeigneten Trägerstoffen und/oder Verteilungsmitteln, gegebenenfalls unter Zusatz von gegenüber den Wirkstoffen inerten Antischaum-, Netz-, Dispersions- und/oder Lösungsmitteln. Die Wirkstoffe können in den folgenden Aufarbeitungsformen vorliegen und angewendet werden:

Feste Aufarbeitungsformen: Stäubemittel, Streumittel, Granulate, Umhüllungsgranulate, Imprägniergranulate und Homogengranulate.

In Wasser dispergierbare Wirkstoffkonzentrate:

Spritzpulver (wettable powder), Pasten, Emulsionen; Emulsionskonzentrate.

Flüssige Aufarbeitungsformen: Lösungen.

Die Wirkstoffkonzentrationen betragen in den erfindungsgemässen Mitteln 1 bis 80 Gew.% und können gegebenenfalls bei der Anwendung auch in geringen Konzentrationen wie etwa 0,05 bis 1% vorliegen.

Den beschriebenen erfindungsgemässen Mitteln lassen sich andere biozide Wirkstoffe oder Mittel beimischen. So können die neuen Mittel ausser den genannten Verbindungen der allgemeinen Formel I, z.B. Insektizide, Fungizide, Bakterizide, Fungistatika, Bakteriostatika, Nematozide oder weitere Herbizide zur Verbreiterung des Wirkungsspektrums, enthalten.

*Spritzpulver*

Zur Herstellung eines a) 70%igen und b) 10%igen Spritzpulvers werden folgende Bestandteile verwendet:

a) 70 Teile Wirkstoff der Formel I
   5 Teile Natriumdibutylnaphthylsulfonat
   3 Teile Naphthalinsulfonsäuren/Phenolsulfonsäuren/Formaldehyd-Kondensat 3:2:1
   10 Teile Kaolin
   12 Teile Champagne-Kreide

b) 10 Teile Wirkstoff
   3 Teile Gemisch der Natriumsalze von gesättigten Fettalkoholsulfaten
   5 Teile Naphthalinsulfonsäuren/Formaldehyd-Kondensat
   82 Teile Kaolin

Der angegebene Wirkstoff wird auf die entsprechenden Trägerstoffe (Kaolin und Kreide) aufgezogen und anschliessend vermischt und vermahlen mit den übrigen Bestandteilen. Man erhält Spritzpulver von vorzüglicher Benetzbarkeit und Schwebefähigkeit. Aus solchen Spritzpulvern können durch Verdünnen mit Wasser Suspensionen von 0,1-8% Wirkstoff erhalten werden, die sich zur Unkrautbekämpfung in Pflanzenkulturen eignen.

*Paste*

Zur Herstellung einer 45%igen Paste werden folgende Stoffe verwendet:

45 Teile Wirkstoff der Formel I
5 Teile Natriumaluminiumsilikat
14 Teile Cetylpolyglykoläther mit 8 mol Äthylenoxid
1 Teil Oleylpolyglykoläther mit 5 mol Äthylenoxid
2 Teile Spindelöl
10 Teile Polyäthylenglykol
23 Teile Wasser

Der Wirkstoff wird mit den Zuschlagstoffen in dazu geeigneten Geräten innig vermischt und vermahlen. Man erhält eine Paste, aus der sich durch Verdünnen mit Wasser Suspensionen jeder gewünschten Konzentration herstellen lassen.

*Emulsionskonzentrat*

Zur Herstellung eines 25%igen Emulsionskonzentrates werden
25 Teile Wirkstoff der Formel I
10 Teile einer Mischung von Nonylphenolpolyoxyäthylen oder Calciumdidecylbenzolsulfonat
10 Teile Cyclohexanon
55 Teile Xylol
miteinander vermischt. Dieses Konzentrat kann mit Wasser zu Emulsionen auf geeignete Konzentrationen von z.B. 0,1% verdünnt werden. Solche Emulsionen eignen sich zur Bekämpfung von Unkräutern in Kulturpflanzungen.

Zum Nachweis der Brauchbarkeit als Herbizide (pre- und postemergent) und zum Beweis der Überlegenheit gegenüber bekannten Wirkstoffen ähnlicher Struktur dienen folgende Testmethoden:

*Preemergente Herbizidwirkung (Keimhemmung)*

Im Gewächshaus wird unmittelbar nach der Einsaat der Versuchspflanzen in Saatschalen die Erdoberfläche mit einer wässerigen Dispersion der Wirkstoffe, erhalten aus einem 25%igen Emulsionskonzentrat resp. aus einem 25%igen Spritzpulver mit Wirkstoffen, die wegen ungenügender Löslichkeit nicht als Emulsionskonzentrat hergestellt werden können, behandelt. Es wurden vier verschiedene Konzentrationsreihen angewendet, entsprechend 4, 2, 1 und 0,5 kg Wirksubstanz/ha. Die Saatschalen werden im Gewächshaus bei 22-25°C und 50-70% rel. Luftfeuchtigkeit gehalten und der Versuch nach 3 Wochen ausgewertet und die Resultate nach folgender Notenskala bonitiert:

1 = Pflanzen nicht gekeimt oder total abgestorben
2-3 = sehr starke Wirkung
4-6 = mittlere Wirkung
7-8 = geringe Wirkung
9 = keine Wirkung (wie unbehandelte Kontrolle)

Als Versuchspflanzen dienen:

| | |
|---|---|
| *Hordeum* (Gerste) | *Setatia italica* |
| *Triticum* (Weizen) | *Echinochloa crus galli* |
| *Zea* (Mais) | *Beta vulgaris* |
| *Sorghum hybridum* (Hirse) | *Sida spinosa* |
| *Oryza* (Reis) | *Sesbania exaltata* |
| *Glycine* (Soja) | *Amaranthus retroflexus* |
| *Gossypium* (Baumwolle) | *Sinapis alba* |
| *Avena fatua* | *Ipomoea purpurea* |
| *Lolium perenne* | *Galium aparine* |
| *Alopecurus myosuroides* | *Pastinaca sativa* |
| *Bromus tectorum* | *Rumex sp.* |
| *Cyperus esculentus* | *Chrysanthemum leucum* |
| *Rottboellia exaltata* | *Abutilon sp.* |
| *Digitaria sanguinalis* | *Solanum nigrum* |

Die geprüften Verbindungen der Formel I zeigten bei 1 und 2 kg/ha ausgezeichnete Wirkung gegen die breitblättrigen und auch gegen die meisten grasartigen Unkräuter, während Kulturpflanzen, wie Mais und zum Teil auch Weizen, Hirse, Reis, Soja und Baumwolle, nicht oder nur unbedeutend geschädigt wurden.

*Postemergente Herbizidwirkung (Kontaktherbizid)*

Eine grössere Anzahl Unkräuter und Kulturpflanzen, sowohl monokotyle wie dikotyle, wurden nach dem Auflaufen (in 4-bis-6-Blattstadium) mit einer wässerigen Wirkstoffdispersion in Dosierungen von 0,5, 1, 2, und 4 kg Wirksubstanz/ha auf die Pflanzen gespritzt und diese bei 24-26°C und 45-60% relativer Luftfeuchtigkeit gehalten. Mindestens 15 d nach der Behandlung wird der Versuch ausgewertet und das Ergebnis wie im preemergent-Versuch nach derselben Notenskala bonitiert.

Auch in diesem Versuch zeigten die geprüften Verbindungen ausgezeichnete Wirkung bei 1 und 2 kg/ha gegen monokotyle und die meisten grasartigen Unkräuter, wobei die Kulturpflanze Mais, die Getreidearten Gerste, Hirse und Reis wie auch Baumwolle und Soja nicht oder erst mit höheren Aufwandmengen Wirkstoff geschädigt wurden.

*Wuchshemmung bei Gräsern*

In Kunststoffschalen mit Erde/Torf/Sand-Gemisch (6:3:1) wurden Samen der Gräser *Lolium perenne*, *Poa pratensis*, *Festuca ovina* und *Dactylis gromerata* ausgesät und normal bewässert. Die aufgelaufenen Gräser wurden wöchentlich bis auf 4 cm Höhe zurückgeschnitten und 40 d nach der Aussaat und 1 d nach dem letzten Schnitt mit wässerigen Spritzbrühen eines Wirkstoffes der Formel I bespritzt. Die Wirkstoffmenge betrug umgerechnet 0,5 und 2,5 kg Aktivsubstanz/ha. 10 und 21 d nach der Applikation wurde das Wachstum der Gräser beurteilt. Gegenüber der Kontrolle erreichten die geprüften Verbindungen bei 2,5 kg/ha eine durchschnittliche Wuchshemmung von 20%.

## Patentansprüche

1. Neue 1,3,4-Thiadiazolyloxyphenylharnstoffe der Formel I:

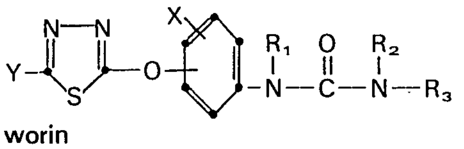

(I)

worin

X Wasserstoff, Halogen oder Trifluormethyl,

Y Wasserstoff, $C_1$ bis $C_6$-Alkyl, gegebenenfalls durch Sauerstoff unterbrochen oder durch Halogen oder Cyan substituiert, $C_3$ bis $C_6$-Cycloalkyl, Phenyl gegebenenfalls substituiert durch Halogen oder $C_1$ bis $C_4$-Alkyl, $C_1$ bis $C_4$-Alkylthio, $C_1$ bis $C_4$-Alkoxy, $C_1$ bis $C_4$-Alkylsulfinyl oder Alkylsulfonyl oder eine Sulfamoylgruppe $-SO_2NR_2R_3$,

$R_1$ Wasserstoff oder $C_1$ bis $C_4$-Alkyl,

$R_2$ Wasserstoff, $C_1$ bis $C_6$-Alkyl, gegebenenfalls durch Sauerstoff unterbrochen oder durch Halogen oder Cyan substituiert, $C_3$ bis $C_6$-Alkenyl oder Alkinyl, $C_3$ bis $C_6$-Cycloalkyl,

$R_3$ dasselbe wie $R_2$ oder $C_1$ bis $C_6$-Alkoxy, oder

$R_2$ und $R_3$ zusammen mit dem Stickstoff, an den sie gebunden sind, auch einen 5-6 gliedrigen Heterocyclus, der noch ein Sauerstoff- oder Schwefelatom oder eine gegebenenfalls durch $C_1$ bis $C_3$-Alkyl substituierte Iminogruppe enthalten kann.

2. Die 1,3,4-Thiadiazolyloxyphenylharnstoffe gemäss Anspruch 1 entsprechend der Formel Ia:

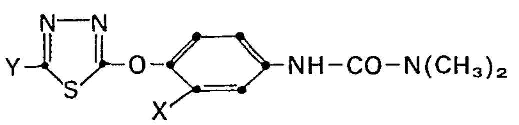

(Ia)

worin X und Y die im Anspruch 1 gegebene Bedeutung haben.

3. Die 1,3,4-Thiadiazolyloxyphenylharnstoffe gemäss Anspruch 1 entsprechend der Formel Ib:

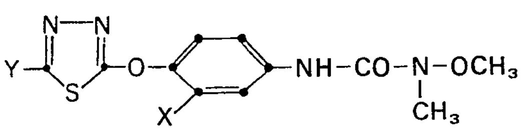

(Ib)

worin X und Y die im Anspruch 1 gegebene Bedeutung haben.

4. Die 1,3,4-Thiadiazolyloxyphenylharnstoffe gemäss Anspruch 1 entsprechend der Formel Ic:

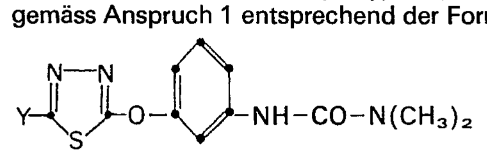

(Ic)

worin Y die im Anspruch 1 gegebene Bedeutung hat.

5. Die 1,3,4-Thiadiazolyloxyphenylharnstoffe gemäss Anspruch 1 der Formel Id:

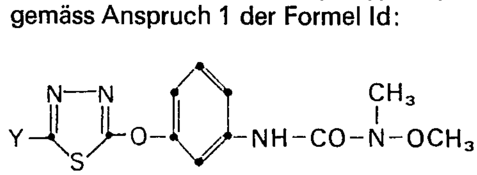

(Id)

worin Y die im Anspruch 1 gegebene Bedeutung hat.

6. Die 1,3,4-Thiadiazolyloxyphenylharnstoffe gemäss Anspruch 1 der Formel I, in denen

X Wasserstoff, Chlor oder Trifluormethyl,

Y Trifluormethyl, Isopropyl, Isobutyl, sek.-Butyl, tert.-Butyl, Isoamyl, Cyclopropyl, Cyclopropylmethyl, Methoxymethyl, 1-Cyanopropyl, Methylsulfonyl, Isopropylsulfonyl oder Dimethylsulfamoyl,

$R_1$ Wasserstoff,

$R_2$ Methyl, und

$R_3$ Methyl oder Methoxy darstellen.

7. N-[4-(2'-Trifluormethyl-1',3',4'-thiadiazolyl-5'-oxy)phenyl]-N',N'-dimethylharnstoff gemäss Anspruch 1.

8. N-[3-(2'-Trifluormethyl-1',3',4'-thiadiazolyl-5'-oxy)phenyl]-N',N'-dimethylharnstoff gemäss Anspruch 1.

9. N-[3-Chlor-4-(2'-trifluormethyl-1',3',4'-thiadiazolyl-5'-oxy)phenyl]-N',N'-dimethylharnstoff gemäss Anspruch 1.

10. N-[4-(2'-Trifluormethyl-1',3',4'-thiadiazolyl-5'-oxy)phenyl]-N'-methyl-N'-methoxyharnstoff gemäss Anspruch 1.

11. N-[3-(2'-Trifluormethyl-1',3',4'-thiadiazolyl-5'-oxy)phenyl]-N'-methyl-N'-methoxyharnstoff gemäss Anspruch 1.

12. N-[4-(2'-tert.-Butyl-1',3',4'-thiadiazolyl-5'-oxy)phenyl]-N',N'-dimethylharnstoff gemäss Anspruch 1.

13. N-[3-Chlor-4-(2'-tert.-butyl-1',3',4'-thiadiazolyl-5'-oxy)phenyl]-N',N'-dimethylharnstoff gemäss Anspruch 1.

14. N-[3-Chlor-4-(2'-tert.-butyl-1',3',4'-thiadiazolyl-5'-oxy)phenyl]-N'-methyl-N'-methoxyharnstoff gemäss Anspruch 1.

15. N-[3-(2'-tert.-Butyl-1',3',4'-thiadiazolyl-5'-oxy)phenyl]-N',N'-dimethylharnstoff gemäss Anspruch 1.

16. N-[3-(2'-tert.-Butyl-1',3',4'-thiadiazolyl-5'-oxy)phenyl]-N'-methyl-N'-methoxyharnstoff gemäss Anspruch 1.

17. N-[4-(2'-Isopropyl-1',3',4'-thiadiazolyl-5'-oxy)phenyl]-N',N'-dimethylharnstoff gemäss Anspruch 1.

18. N-[4-(2'-Isopropyl-1',3',4'-thiadiazolyl-5'-oxy)phenyl]-N'-methyl-N'-methoxyharnstoff gemäss Anspruch 1.

19. N-[3-(2'-Isopropyl-1',3',4'-thiadiazolyl-5'-oxy)phenyl]-N',N'-dimethylharnstoff gemäss Anspruch 1.

20. N-[3-(2'-Isopropyl-1',3',4'-thiadiazolyl-5'-oxy)phenyl]-N'-methyl-N'-methoxyharnstoff gemäss Anspruch 1.

21. Verfahren zur Herstellung der 1,3,4-Thiadiazolyloxyphenylharnstoffe der Formel I, dadurch gekennzeichnet, dass man in einem den Reaktionsteilnehmern gegenüber inerten organischen Lösungsmittel, in Gegenwart der säurebindenden Menge einer Base, einen Hydroxyphenylharnstoff der Formel II:

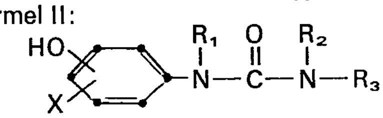

(II)

worin X, $R_1$, $R_2$ und $R_3$ die unter Formel I (Anspruch 1) gegebene Bedeutung haben, mit einem 2-Halogen-1,3,4-thiadiazolylderivat der Formel III umsetzt,

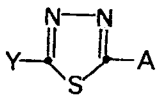 (III)

worin A Halogen oder einen $C_1$ bis $C_4$-Alkylsulfonylrest bedeutet und Y die unter Formel I (Anspruch 1) gegebene Bedeutung hat.

22. Verfahren zur Herstellung der 1,3,4-Thiadiazolyloxyphenylharnstoffe der Formel I (Anspruch 1), dadurch gekennzeichnet, dass man ein 1,3,4-Thiadiazolyloxyanilid der Formel IV:

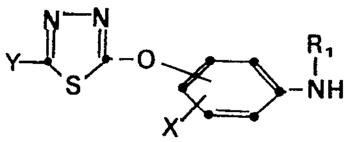 (IV)

worin X, Y und $R_1$ die unter Formel I (Anspruch 1) gegebene Bedeutung haben, in einem den Reaktionspartnern gegenüber inerten organischen Lösungsmittel, in Gegenwart der säurebindenden Menge einer Base, mit einem Carbamoylsäurehalogenid der Formel V umsetzt,

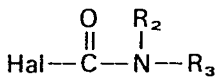 (V)

worin Hal Halogen bedeutet und $R_2$ und $R_3$ die unter Formel I (Anspruch 1) gegebene Bedeutung haben.

23. Verfahren zur Herstellung derjenigen 1,3,4-Thiadiazolyloxyphenylharnstoffe der Formel I, in denen $R_2$ Wasserstoff bedeutet, dadurch gekennzeichnet, dass man ein 1,3,4-Thiadiazolyloxyphenylisocyanat der Formel VI:

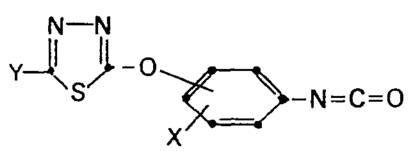 (VI)

worin X und Y die unter Formel I (Anspruch 1) gegebene Bedeutung haben, in einem den Reaktionsteilnehmern gegenüber inerten Lösungsmittel mit einem Amin der Formel VII umsetzt,

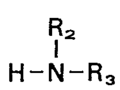 (VII)

worin $R_2$ und $R_3$ die unter Formel I (Anspruch 1) gegebene Bedeutung haben.

24. Herbizides Mittel, dadurch gekennzeichnet, dass es als Wirkstoff mindestens einen 1,3,4-Thiadiazolyloxyphenylharnstoff der Formel I (Anspruch 1) enthält.

25. Die Verwendung der 1,3,4-Thiadiazolyloxyphenylharnstoffe der Formel I (Anspruch 1) oder sie enthaltender Mittel als Herbizide.

26. Die Verwendung der 1,3,4-Thiadiazolyloxyphenylharnstoffe der Formel I, (Anspruch 1) oder sie enthaltender Mittel zur selektiven Unkrautbekämpfung in Kulturen von Nutzpflanzen.

## Claims

1. A 1,3,4-thiadiazolyloxyphenylurea of the formula I:

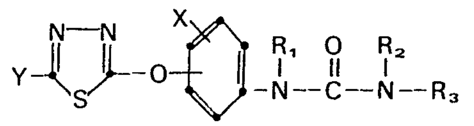 (I)

wherein:

X is hydrogen, halogen or trifluoromethyl,

Y is hydrogen, $C_1$-$C_6$-alkyl which can be interrupted by oxygen or substituted by halogen or cyano, $C_3$-$C_6$-cycloalkyl, phenyl which is unsubstituted or substituted by $C_1$-$C_4$-alkyl; or is $C_1$-$C_4$-alkylthio, $C_1$-$C_4$-alkoxy, $C_1$-$C_4$-alkylsulfinyl or $C_1$-$C_4$-alkylsulfonyl, or a sulfamoyl group $-SO_2NR_2R_3$,

$R_1$ is hydrogen or $C_1$-$C_4$-alkyl,

$R_2$ is hydrogen, $C_1$-$C_6$-alkyl which can be interrupted by oxygen or substituted by halogen or cyano, or is $C_3$-$C_6$-alkenyl or $C_3$-$C_6$-alkynyl, or $C_3$-$C_6$-cycloalkyl,

$R_3$ has the same meaning as $R_2$ or is $C_1$-$C_6$-alkoxy, or

$R_2$ and $R_3$, together with the nitrogen atom to which they are attached, can also form a 5- or 6-membered heterocyclic ring system which can contain an additional oxygen or sulfur atom or an imino group which is unsubstituted or substituted by $C_1$-$C_3$-alkyl.

2. A 1,3,4-thiadiazolyloxyphenylurea according to claim 1 of the formula Ia:

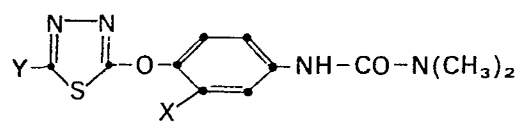 (Ia)

wherein X and Y are as defined in claim 1.

3. A 1,3,4-thiadiazolyloxyphenylurea according to claim 1 of the formula Ib:

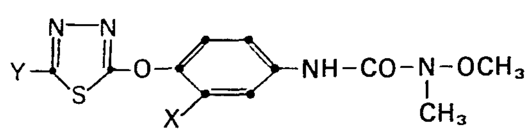 (Ib)

wherein X and Y are as defined in claim 1.

4. A 1,3,4-thiadiazolyloxyphenylurea according to claim 1 of the formula Ic:

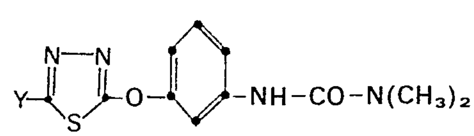 (Ic)

wherein Y is as defined in claim 1.

5. A 1,3,4-thiadiazolyloxyphenylurea according to claim 1 of the formula Id:

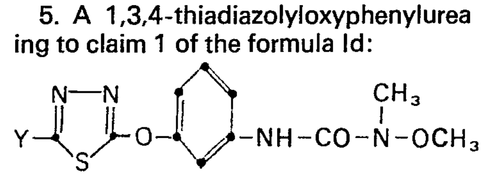 (Id)

wherein Y is as defined in claim 1.

6. A 1,3,4-thiadiazolyloxyphenylurea according to claim 1 of the formula I, wherein X is hydrogen, chlorine or trifluoromethyl, Y is trifluoromethyl, isopropyl, isobutyl, sec.-butyl, tert.-butyl, isoamyl, cyclopropyl, cyclopropylmethyl, methoxymethyl, 1-cyanopropyl, methylsulfonyl, isopropylsulfonyl or dimethylsulfamoyl, $R_1$ is hydrogen, $R_2$ is methyl and $R_3$ is methyl or methoxy.

7. N-[4-(2'-Trifluormethyl-1',3',4'-thiadiazolyl-5'-oxy)phenyl]-N',N'-dimethylurea according to claim 1.

8. N-[3-(2'-Trifluormethyl-1',3',4'-thiadiazolyl-5'-oxy)phenyl]-N',N'-dimethylurea according to claim 1.

9. N-[3-Chloro-4-(2'-trifluormethyl-1',3',4'-thiadiazolyl-5'-oxy)phenyl]-N',N'-dimethylurea according to claim 1.

10. N-[4-(2'-Trifluormethyl-1',3',4'-thiadiazolyl-5'-oxy)phenyl]-N'-methyl-N'-methoxy urea according to claim 1.

11. N-[3-(2'-Trifluormethyl-1',3',4'-thiadiazolyl-5'-oxy)phenyl]-N'-methyl-N'-methoxy urea according to claim 1.

12. N-[4-(2'-tert.-Butyl-1',3',4'-thiadiazolyl-5'-oxy)phenyl]-N',N'-dimethylurea according to claim 1.

13. N-[3-Chloro-4-(2'-tert.-butyl-1',3',4'-thiadiazolyl-5'-oxy)phenyl]-N',N'-dimethylurea according to claim 1.

14. N-[3-Chloro-4-(2'-tert.-butyl-1',3',4'-thiadiazolyl-5'-oxy)phenyl]-N'-methyl-N'-methoxyurea according to claim 1.

15. N-[3-(2'-tert.-Butyl-1',3',4'-thiadiazolyl-5'-oxy)phenyl]-N',N'-dimethylurea according to claim 1.

16. N-[3-(2'-tert.-Butyl-1',3',4'-thiadiazolyl-5'-oxy)phenyl]-N'-methyl-N'-methoxyurea according to claim 1.

17. N-[4-(2'-Isopropyl-1',3',4'-thiadiazolyl-5'-oxy)phenyl]-N',N'-dimethylurea according to claim 1.

18. N-[4-(2'-Isopropyl-1',3',4'-thiadiazolyl-5'-oxy)phenyl]-N'-methyl-N'-methoxyurea according to claim 1.

19. N-[3-(2'-Isopropyl-1',3',4'-thiadiazolyl-5'-oxy)phenyl]-N',N'-dimethylurea according to claim 1.

20. N-[3-(2'-Isopropyl-1',3',4'-thiadiazolyl-5'-oxy)phenyl]-N'-methyl-N'-methoxyurea according to claim 1.

21. A process for the production of a 1,3,4-thiadiazolyloxyphenylurea of the formula I, which process comprises reacting a hydroxyphenylurea of the formula II:

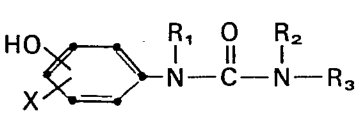
(II)

wherein X, $R_1$, $R_2$ and $R_3$ are as defined for formula I in claim 1, in an inert organic solvent and in the presence of an acid acceptor, with a 2-halogeno-1,3,4-thiadiazolyl derivate of the formula III:

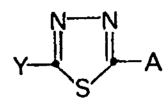
(III)

wherein Y is as defined for formula I in claim 1 and A is halogen or a $C_1$-$C_4$-alkylsulfonyl radical.

22. A process for the production of a 1,3,4-thiadiazolyloxyphenylurea of the formula I according to claim 1, which process comprises reacting a 1,3,4-thiadiazolyloxyanilide of the formula IV:

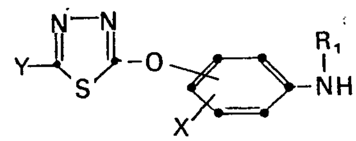
(IV)

wherein X, Y and $R_1$ are as defined for formula I in claim 1, in an inert organic solvent and in the presence of an acid acceptor, with a carbamoyl halide of the formula V:

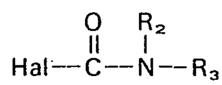
(V)

wherein Hal is halogen and $R_2$ and $R_3$ are as defined for formula I in claim 1.

23. A process for the production of a 1,3,4-thiadiazolyloxyphenylurea of the formula I in which $R_2$ is hydrogen, which process comprises reacting a 1,3,4-thiadiazolyloxyphenylisocyanate of the formula VI:

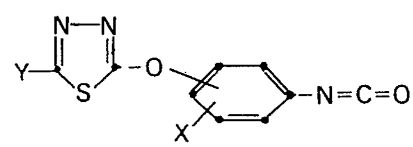
(VI)

wherein X and Y are as defined for formula I in claim 1, in an inert organic solvent, with an amine of the formula VII:

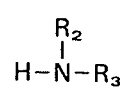
(VII)

wherein $R_2$ and $R_3$ are as defined for formula I in claim 1.

24. A herbicidal composition which contains, as active ingredient, at least one 1,3,4-thiadiazolyloxyphenylurea of the formula I according to claim 1.

25. The use of a 1,3,4-thiadiazolyloxyphenylurea of the formula I according to claim 1, or of a composition containing such a compound, as herbicide.

26. A method of selectively controlling weeds in crops of cultivated plants which comprises, applying to said crops, a herbicidally effective amount of a 1,3,4-thiadiazolyloxyphenylurea of the formula I according to claim 1, or of a composition containing such a compound.

## Revendications

1. Nouvelles 1,3,4-thiadiazolyloxyphénylurées de formule I:

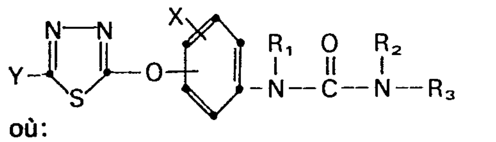 (I)

où:

X représente un hydrogène, un halogène, ou un trifluorométhyle,

Y représente un hydrogène, un alcoyle en $C_1$ à $C_6$ éventuellement interrompu par un oxygène ou substitué par un halogène ou un cyano, un cycloalcoyle en $C_3$ à $C_6$, un phényle éventuellement substitué par un halogène ou un alcoyle en $C_1$ à $C_4$, un alcoylthio en $C_1$ à $C_4$, un alcoxy en $C_1$ à $C_4$, un alcoylsulfinyle en $C_1$ à $C_4$ ou un alcoylsulfonyle en $C_1$ à $C_4$ ou un groupe sulfamoyle $-SO_2NR_2R_3$,

$R_1$ représente un hydrogène ou un alcoyle en $C_1$ à $C_4$,

$R_2$ représente un hydrogène, un alcoyle en $C_1$ à $C_6$ éventuellement interrompu par un oxygène ou substitué par un halogène ou un cyano, un alcényle ou un alcynyle en $C_3$ à $C_6$, un cycloalcoyle en $C_3$ à $C_6$,

$R_3$ a la même signification que $R_2$ ou représente un alcoxy en $C_1$ à $C_6$, ou

$R_2$ et $R_3$ représentent également, avec l'atome d'azote auquel ils sont liés, un hétérocycle à 5 ou 6 chaînons qui peut encore contenir un atome d'oxygène ou de soufre ou un groupe imino éventuellement substitué par un alcoyle en $C_1$ à $C_3$.

2. Les 1,3,4-thiadiazolyloxyphénylurées selon la revendication 1 correspondant à la formule Ia:

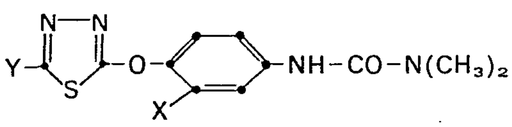 (Ia)

où X et Y ont la signification donnée dans la revendication 1.

3. Les 1,3,4-thiadiazolyloxyphénylurées selon la revendication 1 correspondant à la formule Ib:

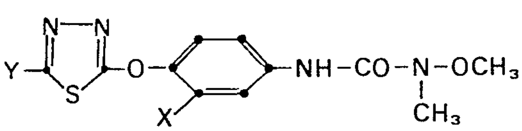 (Ib)

où X et Y ont la signification donnée dans la revendication 1.

4. Les 1,3,4-thiadiazolyloxyphénylurées selon la revendication 1 correspondant à la formule Ic:

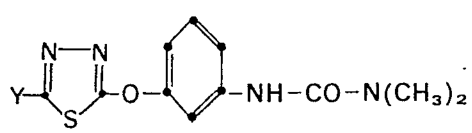 (Ic)

où Y a la signification donnée dans la revendication 1.

5. Les 1,3,4-thiadiazolyloxyphénylurées selon la revendication 1 de formule Id:

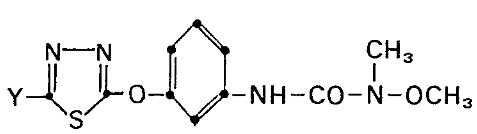 (Id)

où Y a la signification donnée dans la revendication 1.

6. Les 1,3,4-thiadiazolyloxyphénylurées selon la revendication 1 de formule I où:

X représente un hydrogène, un chlore ou un trifluorométhyle,

Y représente un trifluorométhyle, un isopropyle, un isobutyle, un sec.-butyle, un tert.-butyle, un isoamyle, un cyclopropyle, un cyclopropylméthyle, un méthoxyméthyle, un 1-cyanopropyle, un méthylsulfonyle, un isopropylsulfonyle ou un diméthylsulfamoyle,

$R_1$ représente un hydrogène,

$R_2$ représente un méthyle, et

$R_3$ représente un méthyle ou un méthoxy.

7. N-[4-(2'-Trifluorométhyl-1',3',4'-thiadiazolyl-5'-oxy)phényl]-N',N'-diméthylurée selon la revendication 1.

8. N-[3-(2'-Trifluorométhyl-1',3',4'-thiadiazolyl-5'-oxy)phényl]-N',N'-diméthylurée selon la revendication 1.

9. N-[3-Chloro-4-(2'-trifluorométhyl-1',3',4'-thiadiazolyl-5'-oxy)phényl]-N',N'-diméthylurée selon la revendication 1.

10. N-[4-(2'-Trifluorométhyl-1',3',4'-thiadiazolyl-5'-oxy)phényl]-N'-méthyl-N'-méthoxyurée selon la revendication 1.

11. N-[3-(2'-Trifluorométhyl-1',3',4'-thiadiazolyl-5'-oxy)phényl]-N'-méthyl-N'-méthoxyurée selon la revendication 1.

12. N-[4-(2'-tert.-Butyl-1',3',4'-thiadiazolyl-5'-oxy)phényl]-N',N'-diméthylurée selon la revendication 1.

13. N-[3-Chloro-4-(2'-tert.-butyl-1',3',4'-thiadiazolyl-5'-oxy)phényl]-N',N'-diméthylurée selon la revendication 1.

14. N-[3-Chlor-4-(2'-tert.-butyl-1',3',4'-thiadiazolyl-5'-oxy)phényl]-N'-méthyl-N'-méthoxyurée selon la revendication 1.

15. N-[3-(2'-tert.-Butyl-1',3',4'-thiadiazolyl-5'-oxy)phényl]-N',N'-diméthylurée selon la revendication 1.

16. N-[3-(2'-tert.-Butyl-1',3',4'-thiadiazolyl-5'-oxy)phényl]-N'-méthyl-N'-méthoxyurée selon la revendication 1.

17. N-[4-(2'-Isopropyl-1',3',4'-thiadiazolyl-5'-oxy)phényl]-N',N'-diméthylurée selon la revendication 1.

18. N-[4-(2'-Isopropyl-1',3',4'-thiadiazolyl-5'-oxy)phényl]-N'-méthyl-N'-méthoxyurée selon la revendication 1.

19. N-[3-(2'-Isopropyl-1',3',4'-thiadiazolyl-5'-oxy)phényl]-N',N'-diméthylurée selon la revendication 1.

20. N-[3-(2'-Isopropyl-1',3',4'-thiadiazolyl-5'-oxy)phényl]-N'-méthyl-N'-méthoxyurée selon la revendication 1.

21. Procédé de préparation des 1,3,4-thiadiazolyloxyphénylurées de formule I, caractérisé en ce qu'on fait réagir dans un solvant organique inerte vis-à-vis des réactifs, en présence d'une quantité d'une base jouant le rôle de liant acide, une hydroxyphénylurée de formule II:

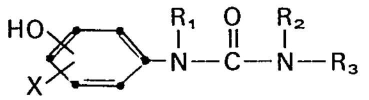

(II)

où X, $R_1$, $R_2$ et $R_3$ ont la signification donnée pour la formule I de la revendication 1, avec un dérivé 2-halogène-1,3,4-thiadiazolyle de formule III:

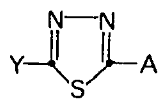

(III)

où A représente un halogène ou un radical alcoyle en $C_1$ à $C_4$ sulfonyle et où Y a la signification donnée pour la formule I de la revendication 1.

22. Procédé de préparation des 1,3,4-thiadiazolyloxyphénylurées de formule I de la revendication 1, caractérisé en ce qu'on fait réagir un 1,3,4-thiadiazolyloxyanilide de formule IV:

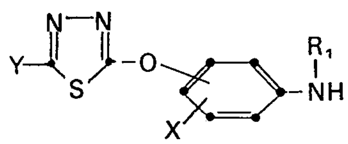

(IV)

où X, Y et $R_1$ ont la signification donnée dans la formule I de la revendication 1, dans un solvant organique inerte vis-à-vis des réactifs, en présence d'une quantité de base jouant le rôle d'agent liant acide, avec un halogénure d'acide carbamoylique de formule V:

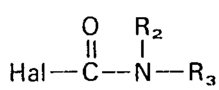

(V)

où Hal représente un halogène et $R_2$ et $R_3$ ont la signification donnée pour la formule I de la revendication 1.

23. Procédé de préparation des 1,2,4-thiadiazolyloxyphénylurées de formule I, où $R_2$ représente un hydrogène, caractérisé en ce qu'on fait réagir un 1,3,4-thiadiazolyloxyphénylisocyanate de formule VI:

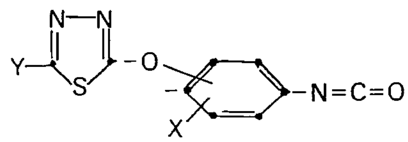

(VI)

où X et Y ont la signification donnée dans la formule I de la revendication 1, dans un solvant inerte vis-à-vis des réactifs, avec une amine de formule VII:

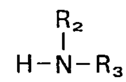

(VII)

où $R_2$ et $R_3$ ont la signification donnée pour la formule I de la revendication 1.

24. Agent herbicide, caractérisé en ce qu'il contient comme matière active au moins une 1,3,4-thiadiazolyloxyphénylurée de formule I de la revendication 1.

25. Application des 1,3,4-thiadiazolyloxyphénylurées de formule I de la revendication 1 ou des agents qui les contiennent comme herbicides.

26. Application des 1,3,4-thiadiazolyloxyphénylurées de formule I de la revendication 1 ou des agents qui les contiennent à la lutte sélective contre les mauvaises herbes dans les cultures de plantes utiles.